Europäisches Patentamt

European Patent Office

Office européen des brevets

(11) Publication number: **0 360 191**
**A2**

## EUROPEAN PATENT APPLICATION

(21) Application number: 89117217.3

(51) Int. Cl.5: **A61K 37/02**

(22) Date of filing: **18.09.89**

(30) Priority: **19.09.88 JP 234380/88**

(43) Date of publication of application:
**28.03.90 Bulletin 90/13**

(84) Designated Contracting States:
**CH DE FR GB IT LI NL**

(71) Applicant: **DAIICHI PHARMACEUTICAL CO., LTD.**
**14-10, Nihonbashi 3-Chome Chuo-Ku Tokyo(JP)**

(72) Inventor: **Nozaki, Junko**
**15-6-306, Shinjuku 6-chome Shinjuku-ku Tokyo(JP)**

(74) Representative: **Kinzebach, Werner, Dr. et al Patentanwälte Reitstötter, Kinzebach und Partner Sternwartstrasse 4 Postfach 86 06 49 D-8000 München 86(DE)**

(54) **Anti-aids virus composition.**

(57) An anti-AIDS virus composition comprising:
(A) a pharmaceutically effective amount of a factor B; and (B) a pharmaceutically acceptable carrier or diluent, and the use of factor B for preparing a pharmaceutical composition for inhibiting the proliferation of an AIDS virus in a human body.

EP 0 360 191 A2

# ANTI-AIDS VIRUS COMPOSITION

## FIELD OF THE INVENTION

This invention relates to an anti-AIDS virus composition containing a factor B as an active ingredient. Furthermore, this invention relates to the use of factor B.

## BACKGROUND OF THE INVENTION

AIDS virus (human immunodeficiency virus. HIV) is a virus infecting human lymphocytes. This virus causes acquired immunodeficiency syndrome, AIDS. Azidothymidine (AZT) and sulfated polysaccharides, among others, are known to be effective against AIDS (Proc. Natl. Acad. Sci. U.S.A., 82, 7096 (1985), Agents Chemother., 30, 933 (1985), European Patent Publication No. 240098A). However, these compounds are not yet fully satisfactory as therapeutic agents because they have adverse effects and/or insufficiently efficacious.

## SUMMARY OF THE INVENTION

Accordingly, it is an object of the present invention to provide an anti-AIDS virus composition which overcomes the problems mentioned above.

It has been found in the present invention that a factor B, which is one of the serum components, has anti-AIDS virus activity.

Thus, the present invention provides an anti-AIDS virus composition comprising: (A) a pharmaceutically effective amount of a factor B; and (B) a pharmaceutically acceptable carrier or diluent.

Also, the present invention provides a method for inhibiting a proliferation of an AIDS virus in a human body which comprises administering a factor B to a human infected with an AIDS virus.

## DETAILED DESCRIPTION OF THE INVENTION

The factor B is, as mentioned above, one of the serum components and occurs in serum normally at a concentration of 200 to 400 μg/ml. In the case of human factor B, the factor is a glycoprotein having a molecular weight of about 90,000 to 93,000 and a carbohydrate content of about 10%. This factor is involved in the alternative pathway of complements which leads to non-specific immune reactions. The activity of this factor in the complement system (e.g., Essential Immunology, 6th Edition, pages 7-9 and 15-17 (1988)) is generally unstable to heat. Thus, for example, the activity is lost upon heating at about 65°C for about 30 minutes. The amino acid sequence of human factor B is reported in EMBO Journal, 3, 153-157 (1984) and Biochem. J., 209, 51-60 (1983). However, it is to be noted that any modification thereof, for example, (i) one in which a part of the amino acid sequence is missing or replaced with some other amino acid or amino acid sequence, (ii) one containing one or more additional amino acids, (iii) one in which the carbohydrate portion is missing or a part of the carbohydrate sequence is missing or replaced with some other carbohydrate or carbohydrate sequence, (iv) one containing one or more additional carbohydrates in a carbohydrate chain, or (v) one having an additional carbohydrate chain or chains, all fall within the scope of the present invention, provided that the resulting modification does not destroy factor B activity in the complement system. Factor B species derived from animals other than humans, inclusive of modifications thereof as mentioned above, also fall within the scope of the present invention, provided that they exhibit the same activity as that of human factor B. However, human-derived factor B is generally more desirable from the viewpoint of adverse effects.

Since, as mentioned above, the factor B is a serum component, the use thereof does not substantially present any serious safety problems. This can be evidenced in an experiment in which no cytotoxicity was observed when lymphocytes were cultured in a medium supplemented with 100 μg/ml of human-derived factor B.

The factor B used in the present invention is administered generally by the intravenous route at a daily dose within the range of 2-1,000 mg per human adult (body weight, about 60 kg). The dose may be increased or decreased depending on the patient's symptoms.

The factor B, together with additives such as a stabilizer (e.g., albumin, sugar or amino acid) and an isotonizing agent, can be made up into an injectable solution by using known pharmaceutical techniques (Refrigeration, 56, 650, 37 (1981)).

The factor B has proliferation inhibiting and inactivating effects upon the AIDS virus and therefore can serve as an excellent anti-AIDS virus agent. Thus, the factor B can be useful as prophylactic agent for inhibiting the onset of an AIDS or AIDS related complex by administering the factor B to carriers of the AIDS virus, and as a prophylactic agent for inhibiting the onset of an AIDS by administering the factor B to the patients of the AIDS related complex. Furthermore, the factor B is useful as a therapeutic agent for the AIDS or AIDS related complex. Factor B is administered in an amount effective to inhibit the replication of the AIDS virus or in a prophylactically or therapeutically effective amount.

The following examples are further illustrative of the present invention but by no means limitative of the scope thereof.

### EXAMPLE 1

#### Materials

1) The AIDS virus strain LAVI was used as the virus and CEM/C113 cells as the host cells.

2) RPMI 1640 medium (manufactured by Gibco) supplemented with 10% (v/v) inactivated fetal calf serum was used as the culture medium.

3) Fresh human serum used was a home-made preparation. Factor B and factor B-deficient human serum were purchased from Sigma Chemical Company. Factor B was dialyzed in advance against magnesium ion- and calcium ion-free 10 mM phosphate buffer (pH: 7.2, hereinafter referred to as PBS(-) cooled in ice water for 12 hours. Then, the factor B concentration was adjusted to 1.0 mg/ml with PBS(-).

#### Method

Host cells precultured for 3 days were challenged with the AIDS virus at an MOI (multiplicity of infection) of 0.05 to 0.1. After an adsorption period of 90 minutes, the cells were suspended in the culture medium at a concentration of $5 \times 10^5$ cells/ml. A 0.5 ml portion of the suspension, 0.4 ml of the culture medium and 0.1 ml of the test solution were poured into one well on a 24-well multiplate. Other wells were filled in the same manner. Control wells contained 0.1 ml of untreated fetal calf serum or the culture medium in lieu of the test solution. Incubation was carried out in 5% $CO_2$-90% air. The percent cell viability determination was performed by the Trypan blue exclusion technique. The AIDS virus antigen positive cell percentage (IFA positive rate) determination was performed by the indirect fluorescent antibody (IFA) technique at timed intervals.

#### Results

1) The results obtained by using, as the test solution, the factor B-deficient serum (test solution A) or the factor B-deficient serum supplemented with factor B (factor B addition level: 25 μg/ml; test solution B) are shown in Table 1 below.

TABLE 1

| Test Solution | Days of Incubation after Infection | Cell Viability | IFA Positive Rate |
|---|---|---|---|
| | | (%) | (%) |
| A | 4 | 28.0 | 95.0 |
| | 7 | 27.7 | 71.4 |
| | 10 | 10.9 | 75.0 |
| | 14 | 3.6 | -- |
| B | 4 | 37.1 | 98.0 |
| | 7 | 37.7 | 73.1 |
| | 10 | 61.0 | 20.0 |
| | 14 | 89.4 | -- |
| C | 4 | 56.5 | 95.0 |
| | 7 | 34.7 | 86.6 |
| | 10 | 22.6 | 80.0 |
| | 14 | 9.1 | -- |

As is evident from the data shown in Table 1 above, the cell viability decreased and the IFA positive rate did not significantly decrease when the factor B-deficient serum was added to the culture medium, like in the control wells. On the other hand, when the factor B was added to the serum, the decrease in cell viability was inhibited and the IFA positive rate decreased. Thus, reproliferation of cells could be demonstrated.

2) The results obtained by using, as the test solution, serum alone (test solution C) or serum supplemented with factor B (factor B addition level: 25 $\mu$g/ml; test solution D) are shown in Table 2 below.

TABLE 2

| Test Solution | Days of Incubation after Infection | Cell Viability | IFA Positive Rate |
|---|---|---|---|
| | | (%) | (%) |
| C | 4 | 69.7 | 73.3 |
| | 7 | 35.1 | 82.5 |
| | 10 | 42.2 | 74.9 |
| | 14 | 37.7 | -- |
| | 21 | 72.2 | 28.5 |
| D | 4 | 61.4 | 96.0 |
| | 7 | 38.9 | 76.3 |
| | 10 | 50.0 | 55.6 |
| | 14 | 64.4 | -- |
| | 21 | 89.6 | 9.7 |

As is evident from the data shown in Table 2 above, in the case of solution C, which always retards the time of cell reproliferation following AIDS virus infection, decreases were found in properdin activity, indicating decreases in the alternative complement pathway function. The addition of factor B to the serum could thus hasten the time of cell reproliferation.

3) The results obtained by using, as the test solution, culture medium supplemented with factor B (factor B addition level: 12.5 $\mu$g/ml; test solution E) are shown in Table 3 below.

4

TABLE 3

| Test Solution | Days of Incubation after Infection | Cell Viability | IFA Positive Rate |
|---|---|---|---|
| | | (%) | (%) |
| E | 4 | 57.9 | 87.5 |
| | 7 | 43.3 | 78.7 |
| | 10 | 69.2 | 33.3 |
| | 14 | 55.4 | -- |
| Control | 4 | 59.0 | 92.4 |
| | 7 | 23.8 | 85.0 |
| | 10 | 34.8 | 91.4 |
| | 14 | 6.4 | -- |

As is evident from the data shown in Table 3 above, factor B added to the medium suppressed the decrease in cell viability and lowered the IFA positive cell rate. Thus, the addition of factor B caused reproliferation of cells.

On the basis of the above results, it was confirmed that factor B has a proliferation inhibiting effect on the AIDS virus.

## EXAMPLE 2

### Materials

The same materials as used in Example 1 were used.

### Method

The factor B solution (0.2 ml) and 0.2 ml of an AIDS virus suspension ($5 \times 10^6$ $TCID_{50}$/ml) were mixed together and the mixture was allowed to stand at $37^\circ$ C for 30 minutes and then diluted 10,000-fold with PBS(-) (test solution F). A control solution was prepared by mixing PBS(-) with the viral suspension, followed by dilution, in the same manner as in the test solution preparation. The test solution or control solution (0.1 ml) was brought into contact with $1 \times 10^6$ CEM/C113 cells. After an adsorption period of 90 minutes, the cell concentration was adjusted to $2.5 \times 10^5$ cells/ml. The cell suspension was distributed in 1 ml portions into wells of a 24-well multiplate and incubation was started. The cell viability and IFA positive rate were determined at timed intervals.

### Results

The results obtained are shown in Table 4 below.

TABLE 4

| Test Solution | Days of Incubation after Infection | Cell Viability | IFA Positive Rate |
|---|---|---|---|
| | | (%) | (%) |
| F | 4 | 91.5 | 1.4 |
| | 7 | 89.5 | 3.6 |
| | 11 | 76.6 | 3.9 |
| Control | 4 | 90.7 | 2.5 |
| | 7 | 89.3 | 31.3 |
| | 11 | 24.5 | 90.5 |

The data shown in Table 4 above indicate that, in the controls, the cell viability decreased and the IFA positive rate increased with time or, in other words, cells were infected with the AIDS virus and the virus proliferated in the cells. On the other hand, in the test run in which the AIDS virus was treated in advance with factor B, the decrease in cell viability and the increase in IFA positive rate were suppressed. It was thus confirmed that factor B is, by itself, able to neutralize the infectivity of the AIDS virus and is useful as an anti-AIDS virus agent.

While the invention has been described in detail and with reference to specific embodiments thereof, it will be apparent to one skilled in the art that various changes and modifications can be made therein without departing from the spirit and scope thereof.

**Claims**

1. An anti-AIDS virus composition comprising:
   (A) a pharmaceutically effective amount of a factor B; and
   (B) a pharmaceutically acceptable carrier or diluent.

2. The use of factor B for preparing a pharmaceutical composition for inhibiting proliferation of an AIDS virus in a human body.

3. The use of factor B for preparing a pharmaceutical composition for inhibiting the onset of AIDS or AIDS related complex.

4. The use of factor B for preparing a pharmaceutical composition for inhibiting the onset of AIDS related complex.

5. The use of facor B for preparing a pharmaceutical composition for treating AIDS or AIDS related complex.